# EUROPÄISCHE PATENTSCHRIFT

(11) **EP 2 159 226 B1**
(45) Veröffentlichungstag und Bekanntmachung des Hinweises auf die Patenterteilung: **31.08.2016**
(21) Anmeldenummer: 08753876.5
(22) Anmeldetag: 20.03.2008
(51) Int. Cl.: C07D 473/06, A61P 37/08, A61P 27/14, A61K 31/522

(54) **ANTIHISTAMIN- UND ANTIALLERGIENMITTEL SOWIE VERFAHREN ZU SEINER HERSTELLUNG**
ANTIHISTAMINIC AND ANTIALLERGIC AGENT AND A METHOD FOR THE PRODUCTION THEREOF.
PRODUIT À ACTIVITÉ ANTI-ALLERGIQUE ANTI-HISTAMINIQUE ET PROCÉDÉ DE FABRICATION CORRESPONDANT

(30) Priorität: 29.03.2007 RU 2007111380; 17.03.2008 RU 2008109708
(43) Veröffentlichungstag der Anmeldung: 03.03.2010
(73) Patentinhaber: Joint-stock Company "Obninsk Chemical Pharmaceutical Company", Kaluga region, 249036 (RU)
(72) Erfinder: GLUSHKOV, Robert Georgievich, Moscow, 125047 (RU); JUZHAKOV, Sergei Danilovich, Moscow, 117321 (RU); FOMINOVA, Olga Samuilovna, Moscow, 119454 (RU); SAZONOVA, Nelli Mikhaielovna, Moskovskaya obl., 141090 (RU); DOLGINOVA, Elena Maksovna, Moscow, 119525 (RU); SHORR, Vadim Aleksandrovich, Moscow, 117593 (RU); BOROVKOV, Mikhail Viktorovich, Moscow, 115088 (RU); ASNINA, Valentina Vasilievna, Moscow, 127644 (RU)
(74) Vertreter: Engelhard, Markus
(86) Internationale Anmeldenummer: PCT/RU2008/000166
(87) Internationale Veröffentlichungsnummer: WO 2008/121019

(56) Entgegenhaltungen:
- WO-A1-01/79188
- BE-A- 890 222
- DE-A1- 3 307 395
- SU-A3- 1 240 361
- US-A- 4 548 820
- US-A- 4 710 572
- US-A- 4 716 165
- ABOU-GHARBIA M ET AL: "NEW ANTIHISTAMINES: SUBSTITUTED PIPERAZINE AND PIPERADINE DERIVATIVES AS NOVEL H1-ANTAGONISTS" JOURNAL OF MEDICINAL CHEMISTRY, AMERICAN CHEMICAL SOCIETY, WASHINGTON, US LNKD- DOI:10.1021/JM00020A018, Bd. 38, Nr. 20, 1. September 1995 (1995-09-01), Seiten 4026-4032, XP002925800 ISSN: 0022-2623
- DATABASE CA [Online] XP008114732 Database accession no. (100:68315) & JP 58 148828 A & RN 81995-87-1, 81996-01-2, 81995-83-7, 81995-84-8, 81995-86-0, 81995-85-9, 81995-88-2, 81995-89-3, 81996-93-2

## Beschreibung

Zur Zeit gibt es ein ziemlich umfangreiches Sortiment von Antihistaminika, die in der Medizin als antiallergische Mittel praktisch angewendet werden. Trotzdem stellt die Suche nach neuen Blockern der H-1-Histaminrezeptoren nach wie vor eine hochaktuelle Aufgabe dar. Das hängt damit zusammen, dass die meisten der verwendeten Arzneimittel dieser Klasse gewisse Nachteile aufweisen. Zu diesen Nachteilen zählen z. B.: nur kurz anhaltende Wirkung, Vorhandensein von Nebeneffekten seitens des zentralen Nervensystems usw. (M.D. Mashkovsky, Arzneimittel, Moskau, Novaya Volna, 2005, 15. Auflage, S. 285-297). In diesem Zusammenhang ist die Suche nach besonderen Antihistaminika (antiallergischen Heilmitteln) mit neuer chemischer Struktur, und zwar in der Reihe der Xanthin-Derivate, von besonderem Interesse. Darunter gibt es einige hochreaktive Naturstoffe (Theophyllin, Theobromin, Koffein), die über hochwertige pharmakotherapeutische Eigenschaften verfügen.

WO 01/79188 offenbart antihistamine Verbindungen der allgemeinen Formel I

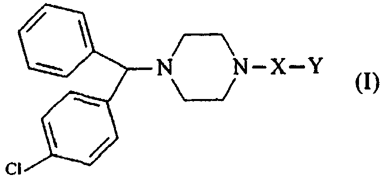

Abou-Gharbia et al., Journal of Medicinal Chemistry, 1995, S. 4026-4032 offenbart substituierte Piperazin- und Piperadin-Derivate als H1-Antagonisten.

US 4,710,572 offenbart N-Theophyllin-7-ylalkylen-N¹-(bis-p-fluorophenyl)-methyl-piperazinderivate als Histamin H₁-Antagonisten.

US 4,548,820 offenbart Xanthin-Verbindungen und deren Verwendung als Bronchodilatierende, anti-allergische und Phosphodiesterase-inhibierende Verbindungen.

DE 33 07 395 A1 offenbart Theobromin- und Theophyllinderivate, die antiphlogistische, antipyretische und analgetische Wirkung haben.

BE 890.222 offenbart Theophyllinderivate und Theobrominderivate mit vasodilatierender oder analgetischer Wirkung.

Das Ziel der Erfindung ist die Erweiterung des Sortiments von antihistaminischen (antiallergischen) Heilmitteln.

Das genannte Ziel wird mittels Synthese und Auswertung der Bioaktivität von besonderen chemischen Verbindungen erreicht, denen die Xanthinstruktur zugrunde liegt. Es handelt sich in diesem Fall insbesondere um die Derivate der 1- und 7-[ω - (Benzhydril-4-piperazinyl-1) alkyl] - 3 - alkylxanthine einschließlich deren Racemate oder optische Isomere sowie ihre pharmazeutisch annehmbaren Salze bzw. ihre Hydrate mit den allgemeinen Formeln I und II: 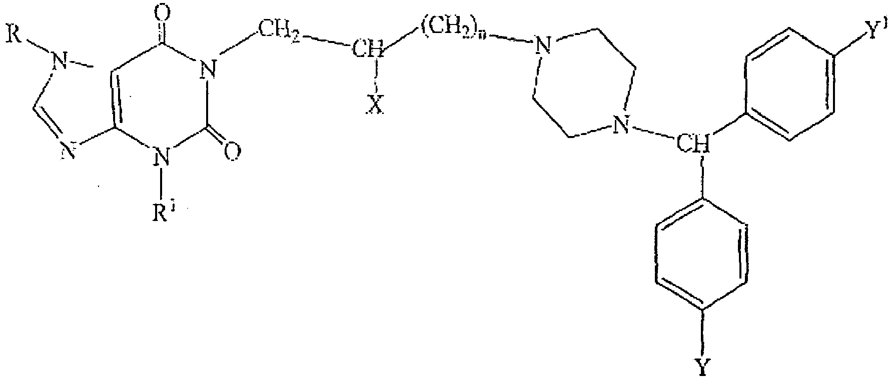 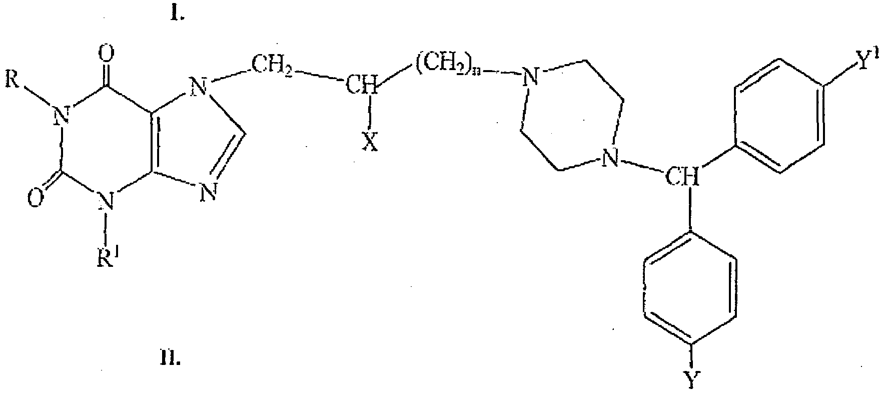 wo R = H, Me, CH₂Ph; R¹ = Me, n - C₄H₉; n = 0-3
X = H, OH, OCOCH₂CH₂COOH; Y und Y¹ = H, Cl, F; die antihistaminisch und antiallergisch wirksam sind und in der Medizin als Basis für die Herstellung von neuen hochreaktiven und wenig toxischen antiallergischen Heilmitteln angewendet werden können.

Die vorliegende Erfindung betrifft die Verbindung 7-[4-(Benzhydrilpiperazinyl-1)butyl]-3-methylxanthin der allgemeinen Formel II: 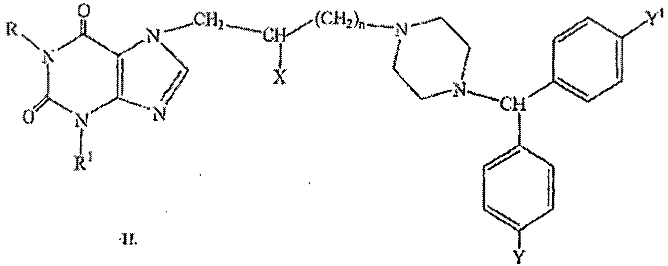 wo R = H; R1 = Me; n = 2
X=H,Y=Y1=H;
sowie ihre pharmazeutisch annehmbaren Salze und/oder ihre Hydrate.

Die angemeldeten Verbindungen und Vergleichsverbindungen werden nach folgenden Verfahren synthetisiert:

### Verfahren A.

Die Xanthin-Derivate der allgemeinen Formeln III und IV, mit R = H, Me, CH₂Ph; R¹⁼ Me, n - C₄H₉; 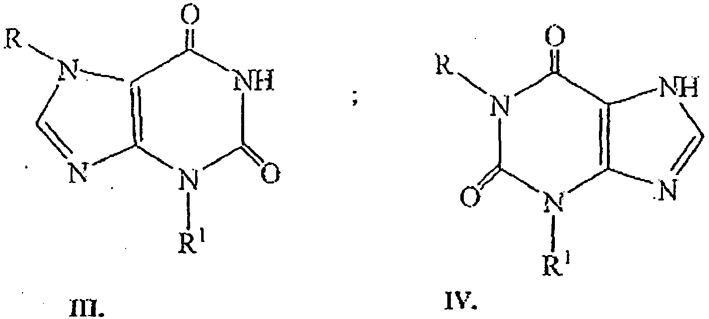 die nach dem klassischen Traube-Verfahren (K.V. Vatsuro, G.L. Mischenko, Namenreaktionen in der organischen Chemie, Moskau, Chimia Verlag, 1976, S. 400; P.M. Kochergin et al., HGS (Die Chemie von heterozyklischen Verbindungen, 1995, Heft 29, S. 388) hergestellt werden, werden in Form von Salz mit einem Alkalimetall (Na, K) mittels α, ω - Dibromalkanen mit der allgemeinen Formel V: Br(CH₂)ₙBr (V, n = 2-5) alkyliert.

Die dadurch gebildeten ω-Bromalkylxanthine der allgemeinen Formeln VI und VII mit R = H, Me, CH₂Ph; R¹= CH₃, n-C₄H₉; n=2-5, 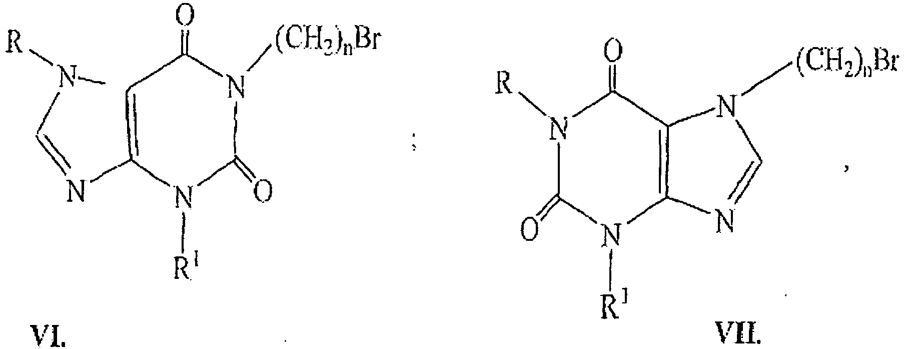 werden mit Jodkalium mittels substituierten Benzhydrilpiperazinen der allgemeinen Formel VIII behandelt: 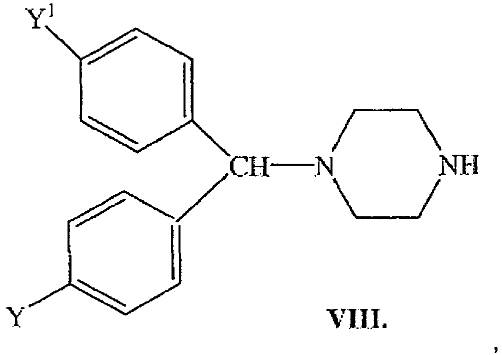 wobei Y und Y¹ = H, Cl, F sind.

Dabei werden die Verbindungen I und II hergestellt, wobei X = H ist (Tabelle 1 ).

### Verfahren B.

Nach diesem Verfahren werden die Xanthinderivate III und IV, mit R = H, Me, CH₂Ph; R¹ = Me, n - C₄H₉; mit Epichlorhydrin behandelt. Dabei werden je nach den Reaktionsbedingungen (wasserfreies Medium oder Wasser) die substituierten 1- oder 7-(2,3-Epoxidpropyl)xanthine (IX und X, mit Z = 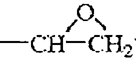 ) oder 1- und 7- (3-Chlor-2-oxypropyl)-xanthine (IX und X, mit Z= - CH(OH)-CH₂Cl) gebildet 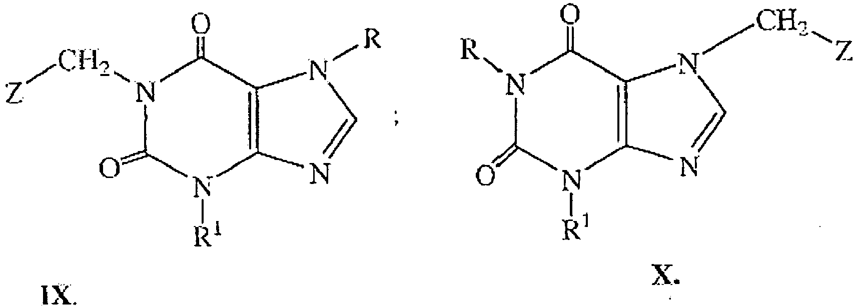 (H.J. Roth, Reaction of theophylline and theobromine with 1,2-epoxides, Arch. Pharm., 1959, 292, 234-238; J. Med. Chem., 1985, 28, Heft 5, 652).

Bei der Zusammenwirkung der Verbindungen IX und X mit Benzhydrilpiperazin und seinen Derivaten werden die Verbindungen I und II hergestellt, mit X = OH; n = 1.

### (Tabelle 1 )

### Die Bioaktivität der Derivate 1- und 7- [w-(Benzhydril-4-Piperazinyl-1)Alkyl]-3-Alkylxanthine (I und II)

Die Forschung nach antihistaminischer Aktivität der angemeldeten Verbindungen wurde nach den bekannten Verfahren (Anleitung für experimentelle Forschung von neuen pharmakologischen Substanzen, Moskau, 2005, S. 489) am abgetrennten Krummdarm der Meerschweinchen (die Unterdrückung des spasmogenen Effekts von Histamin) und an narkotisierten Meerschweinchen (der Antagonismus in Bezug auf Bronchienstriktureffekt von Histamin) vorgenommen. Bei den Versuchen an ganzen Tieren wurden die Substanzen intravenös (5 Minuten vor der Einführung von Histamin) und innerlich (oral) (2 Stunden vor Untersuchungsbeginn) verabreicht.

Für die Auswertung der antihistaminischen Wirkungsdauer der Verbindung wurden die Substanzen 24 bis 72 Stunden vor Beginn der Forschung innerlich verabreicht. Die aktivste Verbindung IIg (mit R = H; R¹ = CH₃; n = 2; X = H; Y = Y¹ = H) wurde ebenfalls an Wachmeerschweinchen anhand des Juck-Modells erforscht. Das Jucken wurde mittels Einträufelung von Histamin (antihistaminische Wirkung) ins Auge des Tiers verursacht. Diese Untersuchung wurde ebenfalls an Ratten anhand des Modells mit passiver Hautanaphylaxie (antiallergischer Effekt) durchgeführt (Anleitung für experimentelle Forschung von neuen pharmakologischen Substanzen, Moskau, 2005 r, S. 511).

Die Verbindungen I und II wurden im Vergleich mit anderen Arzneimitteln wie Cetirizinen, Loratadinen und Azelastinen erforscht, die ihrer pharmakologischen Wirkung nach den Verbindungen I und II ähnlich sind (M.D. Mashkovsky, Arzneimittel, Verlag Novaya Volna, 2005, 15. Auflage, S. 285-297)

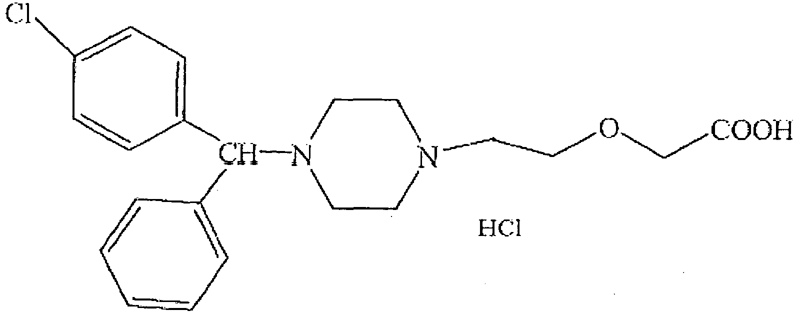

### Cetirizine

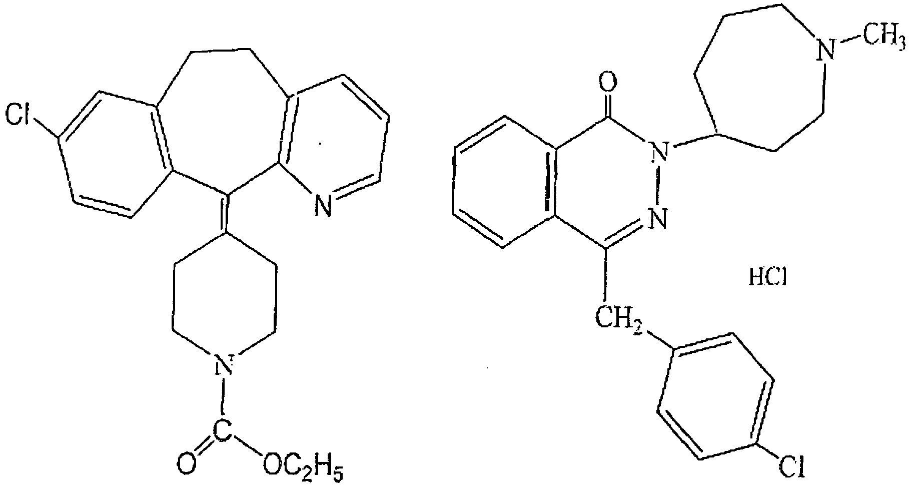

### Loratadine Azelastine

Die Forschungsergebnisse der antihistaminischen Aktivität sowie der Toxizität der Verbindungen I und II sind Tabelle 2 zu entnehmen.

Aus den hier angeführten Angaben folgt, dass die meisten der Verbindungen den Cetirizinen und Loratadinen in vitro und in vivo in ihrer antihistaminischen Aktivität (jeweils die Kennziffern IK₅₀ und ED₅₀) nicht nachstehen. Jedoch sind sie gegenüber Azelastinen weniger aktiv. Eine Ausnahme ist dabei die Verbindung IIg, welche am abgetrennten Darm der Meerschweinchen den Cetirizinen und Loratadinen in ihrer Aktivität wesentlich überlegen ist, wenn auch den Azelastinen etwas nachsteht. Zugleich ist die Verbindung IIg in vivo bei intravenöser Injektion aktiver als alle oben genannten Vergleichspräparate. Bei innerlicher Verabreichung (2 Stunden vor Beginn der Untersuchung) ist die Verbindung IIg in Bezug auf ihre Wirkungskraft den Cetirizinen und Loratadinen überlegen und den Azelastinen ähnlich.

Es sei betont, dass die antihistaminische Wirkungsdauer der Verbindung IIg alle Vergleichspräparate einschließlich der Azelastine übertrifft. So stellen die Verbindungen IIg, Cetirizine, Loratadine und Azelastine beim Modell des Histaminbronchospasmus nach der innerlichen Verabreichung einer Dosis von 3 mg/kg die effektvolle Blockade der H₁-Histaminrezeptoren der Bronchien innerhalb von jeweils 72, 48, 18 und 48 Stunden sicher. Bei dem durch Histamin verursachten Juckmodell wird der Schutzeffekt der Verbindung IIg bei der innerlich verabreichten Dosis von 3 mg/kg stabil innerhalb von 48 Stunden aufrechterhalten. Er lässt erst nach 72 Stunden nach, während die ähnliche Wirkung von Cetirizinen und Azelastinen bei der gleichen Dosis bereits nach 48 Stunden wesentlich abnimmt und nach 72 Stunden vollständig verloren geht. Beim Modell der passiven Hautanaphylaxie weisen die Verbindungen IIg und Cetirizine mit den Dosen von jeweils 1 und 3 mg/kg eine mäßige und ähnliche antiallergische Wirkung auf.

Sehr wichtig ist, dass die Verbindung IIg weniger giftig als Azelastine und Cetirizine ist. Die Verbindung IIg ist hitzestabil und leicht wasserlöslich. Im Unterschied zu den Loratadinen kann sie zur Herstellung von sowohl festen Darreichungsformen als auch Augentropfen angewendet werden.

Aus den erzielten Ergebnissen lässt sich schließen, dass die ermittelte und erforschte Derivatenreihe der 1- und 7- [ω-(Benzhydril-4-piperazinyl-1)alkyl]-3-alkytxanthine (I und II) neue Aussichten in der Behandlung von allergischen Erkrankungen offenbart. Darunter wurden Substanzen mit hoher antihistaminischer und antiallergischer Aktivität entdeckt (Tabelle 2).

Es wird ein Verfahren zur Herstellung von Xanthin-Derivaten, nämlich von 3-Methyl-7-[4-(benzhydril-4-piperazinyl-1)butyl]xanthin und seiner Salze, mit organischen und anorganischen Säuren der allgemeinen Formel **XI** vorgeschlagen, welche antihistaminisch (antiallergisch) aktiv sind.

Die herzustellende Verbindungen **XI** werden aus dem 7-Kaliumsalz von 3-Methylxanthina (XII) mittels Alkylierung unter Einsatz von 1,4-Dibrombutan synthesiert. Danach erfolgt die Zusammenwirkung des gebildeten 7-(4-Brombutyl)-3-methylxanthin (XII) mit 1-Benzhydrilpiperazin **(XIV)** und die Neutralisation der hergestellten Base **XI** durch die organische oder anorganische Säure nach dem folgenden Schema: 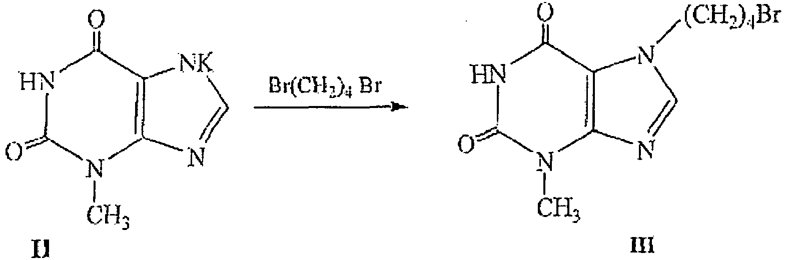 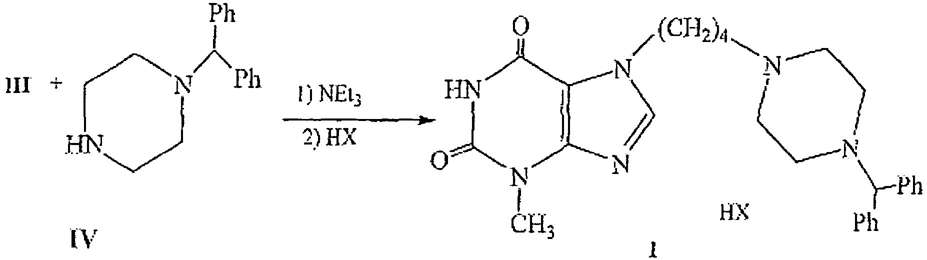 wobei HX organische oder anorganische Säure ist.

**XIV** (1-Benzhydrilpiperazin). und Base **XI** können HBr binden, welches bei der Reaktion von **XIII** mit **XIV** abgeschieden wird. Unter Berücksichtigung dieser Tatsache wurden ursprünglich Ansätze gemacht, **XI** mittels Erwärmung von **XIII** mit **XIV** in verschiedenen Lösungsmitteln zu gewinnen. Jedoch war der Verlauf dieses Prozesses sehr langsam (ca. 30 Stunden) und wurde durch die Bildung von mehreren Nebenprodukten begleitet. Die Versuche, die Reaktion von **XIII** mit **XIV** unter Zugabe von KJ zu katalysieren, führten auch zu keinem positiven Ergebnis.

Der wesentliche Fortschritt in der Vervollkommnung dieser Reaktion wurde durch die Wechselwirkung von **XIII** mit **XIV** unter Erwärmung in Azetonitril mit Triethylamin erreicht. Unter diesen Bedingungen verlief die Gewinnung der **XI**-Base gleichmäßig und innerhalb von 2 bis 3 Stunden. Die Umsetzung der **XI**-Base ins entsprechende Salz erfolgte nach konventionellen Verfahren, welche in der organischen synthetischen Chemie eingesetzt werden, nämlich die Behandlung der Lösung bzw. der Suspension der XI-Base mit der Lösung von organischer oder anorganischer Säure.

Alle gewonnenen Salze (**XI**) waren in ihrer pharmakologischen (antihistaminischen) Aktivität ungefähr gleichwertig. Trotzdem erwies sich **XI** in Form von Salz mit Bernsteinsäure (Succinat) als besonders geeignet für die praktische Anwendung, unter anderem dank der guten pharmakotherapeutischen Eigenschaften (kein Wasseraufnahmevermögen, gute Wärmestabilität, Wasserlöslichkeit und erforderlicher pH-Wert der Wasserlösung).

In Bezug auf die Nutzbarkeit der angemeldeten Verbindungen und insbesondere des Succinats 7-/4-(4-Benzhydrilpiperazinyl-1)butyl/-3-methylxanthin (**XI**, HX - Bernsteinsäure) sei Folgendes betont. Diese Verbindung ist ihren pharmakotherapeutischen Eigenschaften nach (Aktivität und Blockdauer der H₁-Histaminrezeptoren, niedrige Toxizität, Wasserlöslichkeit) den modernen antihistaminischen (antiallergischen) Präparaten Cetirizine, Loratadine und Azelastine überlegen.

### (Nur Beispiele die unter die Ansprüche fallen sind Teil der Erfindung)

### Beispiel 1

### Herstellung von 1-(4-Brombutyl)-3-methyl-7-benzylxanthin (Vlj, R = CH₂Ph; R¹ = CH₃, n = 4).

60 ml abs. CH₃OH, die 2,79 g (0,0517 Mol) von Natriummethylat enthalten, werden mit 10,0 g (0,039 Mol) von 3-Methyl-7-benzylxanthin (III. R = CH₂Ph, R¹ = CH₃) ergänzt.

Die Suspension wird unter Rühren 30 Minuten lang unter Sieden erhitzt. Die Reaktionsmasse wird trocken eingedampft. Am Ende des Ablaufs werden die Methanolreste zusammen mit den Enttoluolen entfernt. Somit werden 10,86 g 1-Natriumsalz 3-Methyl-7-benzylxanthin gewonnen.

Die Lösung von 42,3 g (24,4 ml; 0,196 Mol) 1,4-Dibrombutan in 100 ml Dimethylformamid (DMFA) wird mit 10,86 g (0,039 Mol) 1-Natriumsalz 3-methyl-7-benzylxanthin ergänzt und unter Rühren innerhalb von 3,5 Stunden (bei ca. 150°) unter Sieden erhitzt. Die Masse wird im Vakuum eingedampft. Dem Rest wird Wasser und Benzol zugegeben und das Ganze vermengt. Die Benzolschicht wird abgetrennt, mit Wasser abgespült, über Natriumsulfat und durch eine Schicht von Aluminiumoxid gefiltert. Das Filtrat wird bis auf ca. 30 ml eingedampft und mit der gleichen Menge Hexan ergänzt. Der ausgefällte Rückstand wird gefiltert und getrocknet. Es werden 12,16 g (79,6 %) von 1-(4-Brombutyl)-3-methyl-7-benzylxanthin, Schmelztemperatur 90-92° (aus Methanol), M^{+'}391 hergestellt.

### Beispiel 2

### Herstellung von 3-Methyl-7-(4-Brombutyl)xanthin (VIIj, R = H, R¹ = CH₃, n = 4).

Die Lösung von 42,3 g (0,196 Mol; 23,4 ml) 1,4-Dibrombutan in 100 ml Acetonitril wird mit 10,0 g (0,049 Mol) 7-Kaliumsalz 3-methylxanthin ergänzt. Die Suspension wird unter Rühren innerhalb von 28 Stunden unter Sieden erhitzt und dann gekühlt. Der abgeschiedene Rückstand wird gefiltert, mit Wasser neutral gewaschen, danach mit Methanol gewaschen und getrocknet. So werden 9,0 g (61 %) technisches 3-Methyl-7-(4-brombutyl)xanthin (VII, R = H, R¹ = CH₃, n = 4) nach zweimaliger Reinigung der technischen Probe mittels Kristallisation aus der Mischung von Benzol und Methanol und danach Dimethylsulfoxid, Schmelztemperatur 218-220°, M⁺ 301 hergestellt. Auf ähnliche Weise werden auch andere Verbindungen VI und VII synthesiert (s. Tabelle 3).

### Beispiel 3

Herstellung von 1-(2,3-Epoxidpropyl)-3,7-dimethylxanthin (IX, wo Z= 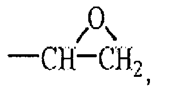 R = R¹ = CH₃).

Die Suspension von 3 g (0,148 Mol) Natriumsalz 3,7-Dimethylxanthin (III, 1-Na Salz; R = R¹ = CH₃) und 20 ml Epichlorhydrin wird unter Rühren bei 65-70° innerhalb von 20 Stunden erwärmt. Nach Abkühlen wird der Rückstand abgefiltert und in Dichlormethan gewaschen. Die hergestellte Lösung IX wird in Dichlormethan eingeengt. Der ölartige Rückstand wird im absoluten Äther vermischt. Somit werden 2,7 g (77 %) 1-(2,3-Epoxidpropyl)-3,7-dimethylxanthin, Schmelztemperatur 115-119°, M^{+'}236 hergestellt (J-C. Pascal et al, J. Med. Chem., 1985, No. 28, No. 5, 647-652, Schmelztemperatur 116-117°).

Ähnlich wird aus Na-Salz 3-Methyl-7-benzylxanthin (III, 1-Na-Salz, R = CH₂Ph; R¹ = CH₃) 1-(2,3-Epoxidpropyl)-3-methyl-7-benzylxanthin (IX, mit Z = 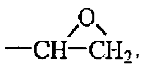 R = CH₂Ph; R¹ = CH₃) synthesiert, Ausbeute 80 %, Schmelztemperatur 90-95°, M^{+'}312.

### Beispiel 4

### Herstellung von 7-(4-Brombutyl)-3-methylxanthin (XIII).

Die Lösung von 42,2 g (0,196 Mol; 23,4 ml) 1,4-Dibrombutan in 100 ml Acetonitril wird mit 10 g (0,049 Mol) 7-Kaliumsalz von 3-Methylxanthin versetzt (M.V. Rubtsov, A.G. Baychikov. Synthetische chemisch-pharmazeutische Präparate. Verlag Medizin. Moskau, 1971, S. 288). Die Suspension wird unter Rühren 28 Stunden unter Sieden erhitzt und dann bis zur Raumtemperatur abgekühlt. Der abgesetzte Rückstand wird abgefiltert, im Wasser neutral gewaschen, danach in Methanol gewaschen und getrocknet. Somit werden 9,0 g (61 %) technisches 7-(4-Brombutyl)-3-methylxanthin nach der Kristallisation aus der Mischung von Benzol-Methanol und danach Dimethylsulfoxid, T_{schm} 218-220 C; M⁺ 301 hergestellt.

### Beispiel 5

### (Endprodukt)

### Herstellung von 7- / 4-(4-Benzhydrilpiperazinyl-1 )butyl/-3- methylxanthin.

50 g der Suspension von 92%iger **XIII,** 43,7 g 97%iger **XIV** und 23,4 ml Triethylamin wurden in 750 ml Acetonitril unter Rühren innerhalb von 3 Stunden unter Sieden erhitzt. Die Reaktionsmasse wird bis zur Raumtemperatur gekühlt und gefiltert. Der Rückstand wird gründlich mit Wasser, Acetonitril und Isopropylalkohol gewaschen und getrocknet. Somit werden 65,9 g der Base 7-/4-(4-Benzhydrilpiperazinyl-1)butyl/-3-methylxanthin, T_{Schm} 193-194 C (aus Methanol) hergestellt.
Berechnet %: C 68,62; H 6,83; N 17,78.

C₂₇H₃₂N₆O₂

Ermittelt %: C 68,41; H 6,93; N 17,78.
NMR-Spektrum (CDCl₃,δ,md): 1,46 (m,2H,γ CH₂); 1,87 (M,2H, β- CH₂); 2,35 (tr.,2H,δ-CH₂); 4,23 (tr.,2H,α-CH₂); 2,15-2,60 (u.sch.s 8H- Protonen des Piperazinzyklus); 3,51 (c,3H, NCH₃); 4,17 (C,1H,CH); 7,10- 7,42 / m,10H, 2(C₆H₅)/; 7,53 (c, 1H, C₈H); 9,00(u.sch.s, 1 H,NH).
Massenspektrum (EU, 70 ev ), m/z: 472 /MT/⁺
Salzsäure, T_{schm} 220-222C (aus 90 % Äthanol); pH der Wasserlösung beträgt 2,45.
Berechnet %: C 59,45; H 6,28; N 15,40

C₂₇H₃₄N₆O₂Cl₂

Ermittelt %: C 58,84; H 6,60; N 15,28; H₂O 2,30
Succinat, T_{schm} 187-189 C (aus 95%-gem Ethanol); pH der Wasserlösung beträgt 4,85.
Berechnet %:C 63,04; H 6,48; N 14,23.

C₃₁H₃₈N₆O₆

Ermittelt %: C 62,88; H 6,74; N 14,17
UV-Spektrum (95%iges Ethanol): ₘₐₓ 273 nm, ₘᵢₙ245 nm
Oxalat, T_{schm} 128-130 C (aus Aceton); pH der Wasserlösung 3,44
Berechnet %: C 61,91; H 6,09; N 14,94

C₂₉H₃₄N₆O₆

Ermittelt %: C 61,45; H 6,83; N 14,94.

### Vergleichsbeispiel 6

Herstellung von 1-[4-(Benzhydril-4-piperazinyl-1)butyl]-3-methylxanthin Dihydrochlorid (I, mit R = H, R¹ = CH₃, X = H, n = 2, Y = Y¹ = H) aus 7-Benzyl-1-(4-brombutyl)-3-methylxanthin (VI, mit R = Benzyl, R¹ = Me).

Die Lösung von 2,1 g (0,005 Mol) von 95%igem 7-Benzyl-1-(4-brombutyl)-3- methylxanthin in 50 ml Methanol und 0,21 g 20%igem Palladiumhydroxid mit Kohlen wird bei 40° bis zum Abschluss der Wasserstoffaufnahme (-1,5 Stunden) hydriert. Der Katalysator wird abgefiltert und mit heißem Methanol gewaschen. Das Filtrat wird eingeengt. Somit werden 1,35 g 1-(4-Brombutyl)-3-methylxanthin (VI, mit R = H, R¹ = Me, n = 4) hergestellt.

Die Mischung aus 1,35 g 1-(4-Brombutyl)-3-methylxanthin, 1,13 g (0,045 Mol) Benzhydrilpiperazin (VIII, mit Y = Y¹ = H), 0,036 g (0,00022 Mol) Jodkalium und 30 ml Acetonitril wird unter Rühren innerhalb von 15 Stunden unter Sieden erhitzt. Die Reaktionsmasse wird eingeengt. Der Rest wird in 25 ml 5%iger Salzsäure aufgelöst. Die Lösung wird mit Chloroform gewaschen. Die Salzsäurelösung wird mit festem Natron bis zum pH von ca. 8 alkalisiert. Die auf diese Weise hergestellte Masse wird mit Chloroform extrahiert. Der Extrakt wird mit gesättigter wässriger Chlornatriumlösung gewaschen und mit Magnesiumsulfat getrocknet.

Die Chloroformlösung wird eingeengt. Der Rest wird in 5 ml Isopropylalkohol aufgelöst. Der Lösung wird gesättigte Lösung von Chlorwasserstoff in Isopropylalkohol bis zum pH von 2 zugegeben. Nach der Abkühlung (5-7°, 16 Stunden) wird der Rückstand abgefiltert und aus der Mischung von Methanol-Isopropylalkohol auskristallisiert. Dadurch werden 1,38 g (50,7 %) von 1-[4-(Benzhydril-4-piperazinyl-1)butyl]-3-methylxanthin Dihydrochlorid, Schmelztempe-ratur 198-198° hergestellt.
Ermittelt %: C59, 41; H6,54; N15,06.

C₂₇H₃₂NeO₂ ^{·} 2 HCl

Berechnet %: C59,45; H6,28; N15,40.

Ähnlich wird aus 1-(4-Brombutyl)-3-butyl-7-benzylxanthin (VI, mit R = CH₂Ph; R¹ = n-Bu; n = 4) und Benzhydrilpiperazin (VIII, mit Y = Y¹ = H) 1-[4-(Benzhydril-4-Piperazinyl-1)butyl]-3-Butylxanthin Dihydrochlorid (I, mit R = H, R¹ = n-Bu, X = H, n = 2, Y = Y¹ = H) mit einer Ausbeute von 42,2 %, Schmelztemperatur 215-217°; M⁺ 514 hergestellt.
Ermittelt %: C60,29; H7,44; N13,93; H₂O 2,55.

C₃₀H₃₈N₆O₂ ^{·} 2 HCl ^{·} 0,75 H₂O

Berechnet %: C59,94; H7,04; N13,98; H₂O 2,25.

### Vergleichsbeispiel 7

### Herstellung von 1,3-Dimethyl-7-[2-oxy-3-(benzhydril-4-piperazinyl-1)propyl]xanthin Dihydrochlorid (II, mit R = R¹ = CH₃, n = 1, X = OH, Y = Y¹ = H)

Die Mischung von 2,45 g (0,009 Mol) 1,3-Dimethyl-7-(2-oxy-3-Chlorpropyl)xanthin (X, mit Z = -CH(OH)-CH₂Cl) (s. H.J. Roth, Arch. Pharm., 1959, 292, 234-238), 2,27 g (0,009 Mol) Benzhydrilpiperazin (VIII, mit Y = Y¹ = H), 1,38 g Pottasche, 0,08 g (0,00005 Mol) Jodkalium und 50 ml Acetonitril wird unter Rühren 20 Stunden lang unter Sieden erhitzt. Die Masse wird eingeengt. Der Rest wird in Chloroform aufgelöst. Die Lösung wird mit Wasser gewaschen und mit Magnesiumsulfat getrocknet. Nach Abziehen des Chloroforms wird der Rest aus dem Ethylacetat auskristallisiert. Dadurch werden 2,61 g der Base II erzeugt (mit R = R¹ = CH₃; n = 1, X = OH, Y= Y¹ = H). Diese Substanz wird in 70 ml Isopropylalkohol aufgelöst. Die resultierende Lösung wird mit der Lösung von Chlorwasserstoff in Isopropylalkohol bis zum pH von ca. 2 versetzt. Der ausgefällte Rückstand wird gefiltert und getrocknet. Das ergibt 3,34 g (66 %) 1,3-Dimethyl-7-[2-oxy-3-(benzhydril-4-piperazinyl-1)propyl]xanthin Dihydrochlorid, Schmelztemperatur 224-226°.
Ermittelt %: C57,32; H6,10; N14,86.

C₂₇H₃₂N₆O₃ ^{·} 2 HCl

Berechnet %: C57,75; H6,10; N14,97.

### Vergleichsbeispiel 8

### Herstellung von 1,3-Dimethyl-7-[2-Carboxyethylcarbonyloxy-3-(benzhydril-4-piperazinyl-1)propyl]xanthin (II, mit R = R¹ = CH₃; n = 1; X = OCOCH₂CH₂COOH; Y = Y¹ = H).

Die Mischung von 2,0 g (0,004 Mol) 1,3-Dimethyl-7-[2-oxy-3(benzhydril-4-piperazinyl-1)propyl]xanthin (II, mit R = R¹ = CH₃; n = 1; X = OH; Y = Y¹ = H), 0,5 g (0,005 Mol) Bernsteinsäureanhydrid und 20 ml Ethylendichlorid wird 4 Stunden lang unter Sieden erhitzt und trocken eingeengt. Der Rest wird aus dem absoluten Ethanol auskristallisiert. Dadurch werden 2,04 g (86,7 %) 1,3-Dimethyl-7-[2-Carboxyethylcarbonyloxy)-3-(benzhydril-4-piperazinyl-1)propyl]xanthin, Schmelztemperatur 183-185° hergestellt. Ermittelt %: C63,28; H6,53; N14,32.

C₃₁H₃₆N₆O₂.

Berechnet %: C63,25; H6,16; N14,28.

### Vergleichsbeispiel 9

### Herstellung von 1-[2-1xy-3-(benzhydril-4-piperazinyl-1)propyl]-3,7-dimethylxanthin Dihydrochlorid I (mit R = R¹ = CH₃; n = 1; X = OH; Y = Y¹ = H).

Die Mischung von 1 g (0,0042 Mol) 1-(2,3-Epoxidpropyl)-3,7-dimethylxanthin, 1,26 g (0,0044 Mol) Benzhydrilpiperazin und 30 ml Isopropylalkohol wird unter Rühren 30 Stunden lang unter Sieden erhitzt. Nach der Abkühlung wird der Niederschlag gefiltert, in Ether gewaschen und getrocknet. Somit werden 1,96 g (95 %) 1-[2-1xy-3-(benzhydril-4-piperazinyl-1) propyl]-3,7- dimethylxanthin hergestellt:
- Base, Schmelztemperatur 120-122°, M^{+·}488;
- Dihydrochlorid, Schmelztemperatur 196-198°; M^{+·}488.

Ähnlich wird Folgendes synthesiert:
- 1-[2-1xy-3-(4¹-chlorbenzhydril-4-piperazinyl-1)propyl]-3,7-dimethylxanthin (I, mit R = R¹=CH₃; n=1; X=OH; Y=Cl; Y¹= H (Vergleichsbeispiel)):
- Base, Ausbeute 67 %, Schmelztemperatur 152-155°;
- Dihydrochlorid, Schmelztemperatur 192-194°.
- 1-[2-1xy-3-(benzhydril-4-piperazinyl-1)-propyl]-3-methyl-7-benzylxanthin (**I**, mit R = CH₂Ph, R¹ = Me; n = 1; X = OH; Y = Y¹ = H),
Ausbeute 50 %, Schmelztemperatur 98-100°.
- 1-[2-1xy-3-(4¹-chlorbenzhydril-4-piperazinyl-1)propyl]-3-methyl-7-benzylxanthin (I, mit R = CH₂Ph, R¹ = Me; n = 1; X = OH; Y = Cl; Y¹ = H),
Ausbeute 50 %, Schmelztemperatur 170-172°.

Diese Substanzen erweitern das Sortiment der antihistaminischen (antiallergischen) Heilmittel mit verlängerter Wirkung. Das vorgeschlagene Verfahren ermöglicht es seinerseits, die Zeit zu reduzieren, welche für die Durchführung des ganzen verfahrenstechnischen Zyklus im Vergleich zu bekannten Verfahren erforderlich ist.

**Tabelle 1.**

| Derivate von 1- und 7-[ω-(Benzhydril-4-piperazinyl-1)alkyl]-3-methylxanthinen (I und II, wo R¹ = CH₃) | | | | | | | | |
|---|---|---|---|---|---|---|---|---|
| Verbindung | n | X | Y | Y¹ | R | Brutto-Formel | Schmelztemperatur, C° | Massenspektrum, EU m/z; M^{+o} |
| 1 | 2 | 3 | 4 | 5 | 6 | 7 | 8 | 9 |
| la | 0 | H | H | H | CH₂Ph | C₃₂H₃₄N₆O₂•2HCl•H₂O | 200-210 | 534 |
| lb | 1 | H | H | H | CH₂Ph | C₃₃H₃₆N₆O₂•2HCl | 187-189 | 548 |
| lc | 1 | H | H | Cl | CH₂Ph | C₃₃H₃₅ClN₆O₂•2HCl•0,5H₂O | 189-192 | 582 |
| ld | 1 | H | F | F | CH₂Ph | C₃₃H₃₄F₂N₆O₂•2HCl | 176-178 | 584 |
| le | 1 | H | H | H | 4-FC₆H₄CH₂ | C₃₃H₃₅FN₆O₂•2HCl | 224-226 | 566 |
| lf | 1 | H | F | F | 4-FC₆H₄CH₂ | C₃₃H₃₃F₃N₆O₂•2HCl | 178-180 | 602 |
| lg | 1 | H | H | H | CH₂CO₂H | C₂₈H₃₂N₆O₄•H₂O | 153-155 | 516 |
| lh | 1 | H | H | H | CH₂CO₂K | C₂₈H₃₁KN₆O₄•1,5H₂O | 146-148 | - |
| li | 2 | H | H | H | CH₂Ph | C₃₄H₃₈N₆O₂•2HCl | 145-146 | 562 |
| lj | 2 | H | H | H | 4-FC₆H₄CH₂ | C₃₄H₃₇FN₆O₂•2HCl | 154-157 | 580 |
| lk | 2 | H | F | F | 4-FC₆H₄CH₂ | C₃₄H₃₅F₃N₆O₂•2HCl | 134-136 | 616 |
| ll | 2 | H | F | F | CH₂Ph | C₃₄H₃₆F₂N₆O₂•2HCl• 1,5H₂O | 171-173 | 598 |
| lm | 3 | H | H | H | CH₂Ph | C₃₅H₄₀N₆O₂•2HCl | 207-208 | 576 |
| ln | 3 | H | F | F | CH₂Ph | C₃₅H₃₈F₂N₆O₂•2HCl | 189-192 | 612 |
| lo | 3 | H | H | H | H | C₂₅H₂₈N₆O₂•2HCl•0,5H₂O | 210-212 | 444 |
| lp | 0 | H | F | F | H | C₂₅H₂₆F₂N₆O₂•HCl | 202-205 | 480 |
| lq | 1 | H | H | H | H | C₂₆H₃₀N₆O2•2HCl•H₂O | 205-207 | 458 |
| lr | 1 | H | F | F | H | C₂₆H₂₈F₂N₆O₂•2HCl | 215-220 | 494 |
| Is | 2 | H | H | H | H | C₂₇H₃₂N₆O₂•2HCl | 196-198 | 472 |
| lt | 2 | H | H | C1 | H | C₂₇H₃₁ClN₆O₂•2HCl•H₂O | 192-194 | 506 |
| lu | 2 | H | F | F | H | C₂₇H₃₀F₂N₆O₂•2HCl•0,5H₂O | 202-206 | 508 |
| lv | 2 | H | H | H | CH₃ | C₂₈H₃₄N₆O₂•2HCl | 171-174 | 486 |
| lw | 3 | H | H | H | H | C₂₈H₃₄N₆O₂•2HCl | 241-242 | 486 |
| lx | 1 | OH | H | H | CH₃ | C₂₇H₃₂N₆O₃•2HCl•2H₂O | 196-198 | 488 |
| ly | 1 | OH | H | Cl | CH₃ | C₂₇H₃₁ClN₆O₃•2HCl• 1,5H₂O | 192-194 | 522 |
| lz | 1 | OH | H | H | CH₂Ph | C₃₃H₃₆N₆O₃ | 98-100 | 564 |
| laa | 1 | OH | H | H | CH₂Ph | C₃₃H₃₅ClN₆O₃ | 170-172 | 598 |
| lla | 0 | H | H | H | CH₃ | C₂₆H₃₀N₆O₂•2HCl | 240-242 | 458 |
| llb | 0 | H | H | C1 | CH₃ | C₂₆H₂₉ClN₆O₂•2HCl | 241-243 | 492 |
| llc | 0 | H | F | F | CH₃ | C₂₆H₂₈F₂N₆O₂•2HCl | 237-238 | 494 |
| lld | 1 | H | H | H | CH₃ | C₂₇H₃₂N₆O₂•2HCl | 238-240 | 472 |
| lle | 1 | H | H | H | H | C₂₆H₃₀N₆O₂•2HCl | 258-260 | 458 |
| llf | 2 | H | H | H | CH₃ | C₂₈H₃₄N₆O₂•2HCl | 224-226 | 486 |
| llg | 2 | H | H | H | H | C₂₇H₃₂N₆O₂•2HCl• 1,5H₂O | 216-217 | 472 |
| llh | 1 | OH | H | H | CH₃ | C₂₇H₃₂N₆O₃•2HCl | 244-246 | 488 |
| lii | 1 | OH | H | Cl | CH₃ | C₂₇H₃₂ClN₆O₃•2HCl•H₂O | 232-234 | 522 |
| llj | 1 | OH | F | F | CH₃ | C₂₇H₃₀F₂N₆O₂•2HCl | 225-227 | 524 |

**Tabelle 2**

| Antihistaminische Aktivität und akute Toxizität von 1- und 7-[ω-(Benzhydril-4-piperazinyl-1)alkyl]-3-methylxanthinen (I und II, mit R¹ = CH₃) | | | | |
|---|---|---|---|---|
| | | | | |
| Verbindung | Antihistaminische Aktivität | | | Akute Toxizität, LD₅₀ mg/kg Mäuse, orale Verabreichung |
| | Abgetrennter Krummdarm der Meerschweinchen, IK₅₀ (M) | Meerschweinchen (*in vivo)* | | |
| | | Intravenöse Injektion ED₅₀, mg/kg | orale Verabreichung ED₅₀, mg/kg | |
| 1 | 2 | 3 | 4 | 5 |
| la | 7,92 ± 2,6·10⁻⁷ | | | > 1000,0 |
| lb | 1,26 ± 0,33·10⁻⁷ | 0,3 | | > 800,0 |
| lc | 1,33 ± 0,13·10⁻⁷ | | | 400,0 |
| ld | 1,14 ± 0,25·10⁻⁷ | 0,6 | 3,0 | > 250,0 |
| le | 4,3 ±0,71·10⁻⁷ | | | |
| lf | 5,14 ± 0,72·10⁻⁷ | | | |
| lg | 2,44 ± 0,73·10⁻⁷ | | | |
| lh | 2,22 ± 0,51·10⁻⁷ | | | >1000,0 |
| li | 1,11 ± 0,12·10⁻⁷ | 0,2 | 0,3 | 400,0 |
| lj | 2,27 ± 0,37·10⁻⁷ | | | 400,0 |
| lk | 2,67 ± 0,18·10⁻⁷ | | | 400,0 |

| 1 | 2 | 3 | 4 | 5 |
|---|---|---|---|---|
| ll | 1,85.± 0,56·10⁻⁷ | 0,5 | 0,5 | >1000,0 |
| lm | 0,92 ± 0,17·10⁻⁷ | | | >200,0 |
| ln | 2,53 ± 0,34·10⁻⁷ | 1,2 | 3,0 | 250 |
| lo | 1,62 ± 0,16·10⁻⁷ | | | >1000,0 |
| lp | 1,92 ± 0,14·10⁻⁷ | 1,5 | | |
| lq | 0,42 ± 0,08·10⁻⁷ | 0,4 | | >1000,0 |
| lr | 0,76 ± 0,15·10⁻⁷ | | >3,0 | >1000,0 |
| ls | 0,26+0,02·10⁻⁷ | 0,2 | 0,3 | >750,0 |
| lt | 1,04 ± 0,29·10⁻⁷ | 0,3 | | >1000,0 |
| lu | 0,51 ± 0,11·10⁻⁷ | 0,7 | >3,0 | >1000,0 |
| lv | 0,32 ± 0,03·10⁻⁷ | 0,2 | >1,5 | >1000,0 |
| lw | 0,74 ± 0,11^{.}10⁻⁷ | 0,1 | | |
| lx | 12,7 ± 3,7·10⁻⁷ | | | |
| ly | 1,23 ± 0,07·10⁻⁷ | | | |
| lz | 5,24 ± 0,06·10⁻⁷ | | | |
| laa | 5,36 ± 1,62·10⁻⁷ | | | |
| lla | 0,14 ± 0,02·10⁻⁷ | 0,1 | | |
| llb | 0,32 ± 0,11·10⁻⁷ | | | >125,0 |
| llc | 0,17 ± 0,04·10⁻⁷ | 0,2 | 8,0 | >1000,0 |
| lld | 0,12 ± 0,02·10⁻⁷ | 0,05 | | >1000,0 |
| lle | 0,19 ± 0,02·10⁻⁷ | 0,1 | | 900,0 |
| llf | 0,17 ± 0,04·10⁻⁷ | 0,1 | <1,5 | 750,0 |
| llg | 0,18 ± 0,04·10⁻⁷ | 0,02 | 0,02 | 1300,0 |
| llh | 1,59 ± 0,22·10⁻⁷ | 0,1 | 2,0 | >1000,0 |
| lli | 2,94 ± 0,73-10⁻⁷ | | | >1000,0 |
| llj | 1,47 ± 0,51·10⁻⁷ | | | >1000,0 |
| Cetirizine | 1,27 ± 0,55·10⁻⁷ | 0,07 | 0,2 | 404,5 |
| Loratadine | 4,72 ± 1,35·10⁻⁷ | 0,3 | 0,9 | 4000,0 |
| Azelastine | 0,07 ± 0,004·10⁻⁷ | 0,05 | 0,015 | 124-139^{**} (^{♂-♀}) |

| | | | | |
|---|---|---|---|---|
| * Dridi D. et al, Circadian time - depended differences in murine tolerance to the antihistaminic agent loratidine, Chronobiol, Inter, 2005, 22 (3), 499-514. ** Zechel H.J. et. al, Pharmacological and lexicological properties of Azelastine, f novel antiallergical agent, Arzneimittelforschung, 1981, 31(8), 1184-1193 | | | | |

**Tabelle 3.**

| Verbindung | n | R | R¹ | Ausbeute, % | Schmelztemperatur, °C | Massenspektrum, EU, m/z; M⁺ |
|---|---|---|---|---|---|---|
| 1 | 2 | 3 | 4 | 5 | 6 | 7 |
| VI a | 2 | CH₂Ph | CH₃ | 65 | 77-79 | 363 |
| VI b | 3 | CH₂Ph | CH₃ | 70 | 81-83 | 377 |
| VI e | 3 | 4-FC₆H₄CH₂ | CH₃ | 66 | 123-125 | 395 |
| VI k | 4 | 4-FC₆H₄CH₂ | CH₃ | 67 | 92-94 | 409 |
| VI n | 5 | CH₂Ph | CH₃ | 59 | 96-98 | 405 |
| VI w | 4 | CH₃ | CH₃ | 60 | 120-122 | 315 |
| VI | 4 | CH₂Ph | H-C₄H₉ | 75 | 88-90 | 433 |
| VII a | 2 | CH₃ | CH₃ | 70 | 143-145 | 287 |
| VII d | 3 | CH₃ | CH₃ | 60 | 131-133 | 301 |
| VII e | 3 | H | CH₃ | 60 | 126-128 | 287 |
| VII j | 4 | CH₃ | CH₃ | 65 | 123-125 | 315 |
| VII | 4 | H | H-C₄H₉ | 59 | 86-88 | 343 |

## Patentansprüche

1. Verbindung 7-[4-(Benzhydrilpiperazinyl-1)butyl]-3-methylxanthin der allgemeinen Formel II: 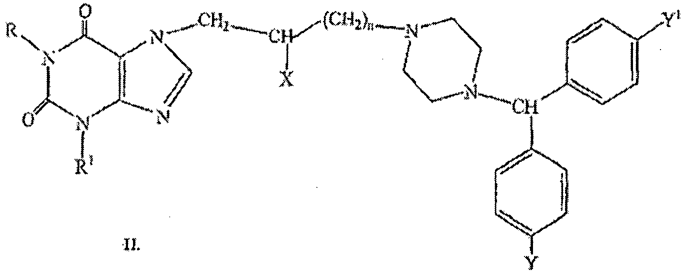 woR=H;R1=Me;n=2
X=H,Y=Y1 =H;
sowie ihre pharmazeutisch annehmbaren Salze und/oder ihre Hydrate.

2. Verbindung nach Anspruch 1, nämlich 7-[4-(Benzhydrilpiperazinyl-l)butyl]-3-methylxanthin Dihydrochlorid, welche eine antihistaminische und antiallergische Wirkung aufweist.

3. Verbindung nach Anspruch 1, nämlich 7-[4-(Benzhydrilpiperazinyl-1)butyl]-3-methylxanthin Succinat, welche eine antihistaminische und antiallergische Wirkung aufweist.

4. Verfahren zur Herstellung von 3-Methyl-7-[4-(benzhydril-4-piperazinyl-I)butyl]xanthin und seiner Salze mit organischen oder anorganischen Säuren der allgemeinen Formel XI: 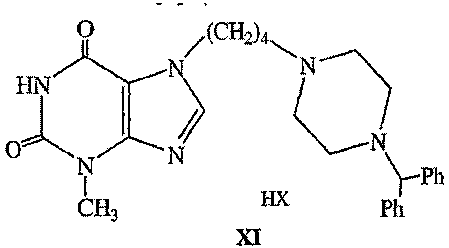 wobei HX eine organische oder anorganische Säure ist, durch Alkylierung des 7-Kaliumsalzes von 3-Methylxanthin mittels 1,4-Dibrombutan mit nachfolgender Zusammenwirkung des gebildeten 7-(4-Brombutyl)-3-Methylxanthin mit 1-Benzhydrilpiperazin und Neutralisation der gebildeten Base von 7-[4-(4-Benzhydrilpiperazinyl-1)butyl]-3-methylxanthin mittels organischer oder anorganischer Säure.

5. Verfahren nach Anspruch 4,
**dadurch gekennzeichnet,**
**dass** der Alkylierungsablauf des 7-Kaliumsalzes von 3-Methylxanthin mittels 1,4-Dibrombutan sowie die Zusammenwirkung von 7-(4-Brombutyl)-3-methylxanthin mit 1-Benzhydrilpiperazin in organischem Lösungsmittel, z. B. in Acetonitril, vorgenommen wird.

6. Verfahren nach den Ansprüchen 4 und 5,
**dadurch gekennzeichnet,**
**dass** die Wechselwirkung von 7-(4-Brombutyl)-3-methylxanthin mit 1-Benzhydrilpiperazin mit einer organischer Base, z.B. Triethylamin, vorgenommen wird.

## Claims

1. A compound 7-[4-(benzhydrylpiperazinyl-1)butyl]-3-methylxanthine of general Formula II and pharmaceutically acceptable salts and/or hydrates thereof: 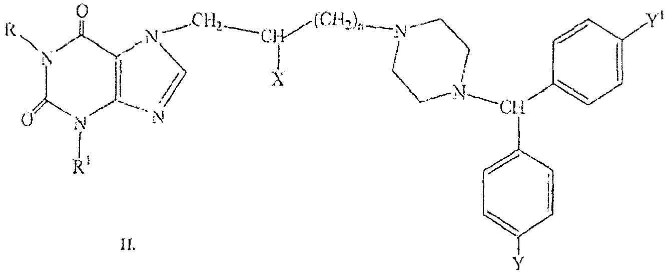 wherein R=H, R¹ = Me, n=2
X=H, Y=Y¹=H,

2. The compound according to claim 1, which is 7-[4-(benzhydrylpiperazinyl-1)butyl]-3-methylxanthine dihydrochloride, capable of producing an antihistaminic and antiallergenic effect.

3. The compound according to claim 1, which is 7-[4-(benzhydrylpiperazinyl-1)butyl]-3-methylxanthine succinate, capable of producing an antihistaminic and antiallergenic effect.

4. Method of producing 7-[4-(benzhydrylpiperazinyl-1)butyl]-3-methylxanthine and salts thereof with an organic acid or inorganic acid of the general formula XI: 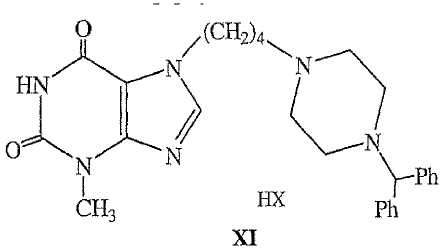 wherein HX is an organic acid or inorganic acid, comprising alkylating the 7-potassium salt of 3-methylxanthine with 1,4-dibrombutane with subsequent interaction of the 7-(4-brombutyl)-3-methylxanthine formed with 1-benzhydrylpiperazine and neutralizing of the produced base, 7-[4-(4-benzhydrylpiperazinyl-1)butyl]-3-methylxanthine, by organic or inorganic acid.

5. Method of producing according to claim 4, wherein alkylating of the 7-potassium salt of 3-methylxanthine with 1,4-dibrombutane, as well as the interaction of 7-(4-brombutyl)-3-methylxanthine with 1-benzhydrylpiperazine, takes place in an organic solvent, e.g., acetonitrile.

6. Method of producing according to claims 4-5, wherein the interaction of 7-(4-brombutyl)-3-methylxanthine with 1-benzhydrylpiperazine takes place in the presence of an organic base, e.g., triethylamine.

## Revendications

1. Un composé 7-[4-(benzhydrylpipérazinyle-1)butyl]-3-méthylxanthine de formule générale II et ses sels pharmaceutiquement acceptables et/ou leurs hydrates: 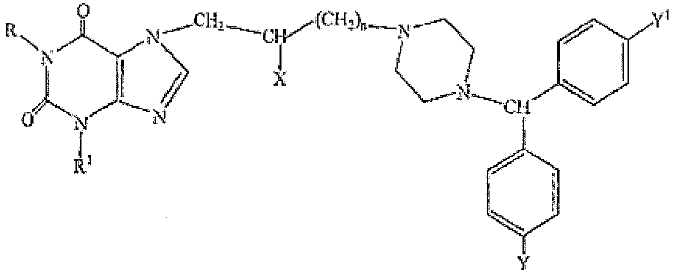 où R=H, R1 = Me, n=2
X=H, Y=Y¹=H.

2. Un composé selon la revendication 1, notamment, le dichlorhydrate de 7-[4-(benzhydrylpipérazinyle-)butyl]-3-méthylxanthine capable de produire un effet antihistaminique et antiallergique.

3. Un composé selon la revendication 1, notamment, le succinate de 7-[4-(benzhydrylpipérazinyle-)butyl]-3-méthylxanthine capable de produire un effet antihistaminique et antiallergique.

4. Procédé de préparation de 7-[4-(benzhydrylpipérazinyle-1)butyle]-3-méthylxanthine et de ses sels avec les acides organiques ou inorganiques de formule générale XI: 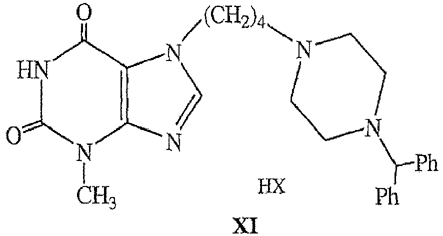 où HX est un acide organique ou inorganique; comprenant une réaction d'alkylation du sel de 7-potassium de 3-méthylxanthine avec 1,4-dibromobutane et une interaction subséquente du 7-(4-bromobutyl)-3-méthylxanthine formé avec 1-benzhydrylpipérazinyle et une réaction de neutralisation de la base produite du 7-[4-(4-benzhydrylpipérazinyle-1)butyl]-3-méthylxanthine avec un acide organique ou inorganique.

5. Procédé de préparation selon la revendication 4, **caractérisé en ce que** la réaction d'alkylation du sel de 7-potassium de 3-méthylxanthine avec 1,4-dibromobutane ainsi que l'interaction du 7-(4-bromobutyl)-3-méthylxanthine avec 1-benzhydrylpipérazinyle s'effectuent dans un solvant organique, par example, l'acétonitrile.

6. Procédé de préparation selon les revendications 4 - 5, **caractérisé en ce que** l'interaction du 7-(4-bromobutyl)-3-méthylxanthine avec 1-benzhydrylpipérazinyle s'effectue en presence d'une base organique, par example, en presence de la triéthylamine.
